Europäisches Patentamt

European Patent Office

Office européen des brevets

(11) Veröffentlichungsnummer: **0 512 499 A1**

## (12) EUROPÄISCHE PATENTANMELDUNG

(21) Anmeldenummer: **92107630.3**

(22) Anmeldetag: **06.05.92**

(51) Int. Cl.5: **A61M 5/32**

(30) Priorität: **08.05.91 DE 4115024**

(43) Veröffentlichungstag der Anmeldung:
**11.11.92 Patentblatt 92/46**

(84) Benannte Vertragsstaaten:
**BE DE DK ES FR GB GR IT LU NL PT**

(71) Anmelder: **HOECHST AKTIENGESELLSCHAFT**
**Postfach 80 03 20**
**W-6230 Frankfurt am Main 80(DE)**

(72) Erfinder: **Pajunk, Horst**
**Am Holzplatz 5-9**
**W-7716 Geisingen(DE)**
Erfinder: **Pajunk, Heinrich**
**Am Holzplatz 5-9**
**W-7716 Geisingen(DE)**
Erfinder: **Loos, Hans-Joachim**
**An der Schleuse 5**
**W-6095 Ginsheim-Gustavsburg(DE)**
Erfinder: **Ziegert, Günter**
**Loreleistrasse 34**
**W-6230 Frankfurt am Main 80(DE)**

(54) **Vorrichtung zum Entsorgen von Injekfionskanülen.**

(57) Bei der Vorrichtung zum Entsorgen von Injektionskanülen, weist ein Gehäuse (1) eine Öffnung (14) auf, in der ein Spannfutter (2) für die Kanülen (11) angeordnet ist. Das Spannfutter (2) ist mit einem Antrieb (3) zum Öffnen und Schließen des Spannfutters (2) verbunden. Das Gehäuse (1) ist mit einer Schublade (5) zur Aufnahme eines Auffangbehälters (6) versehen, in den die Öffnung (14) mündet.

Fig. 1

EP 0 512 499 A1

Injektionskanülen werden manuell vom Spritzenkörper bzw. dem Spritzengestell entfernt und entsorgt. Dabei sind Stichverletzungen und eventuell damit verbundene Infektionen nicht zu vermeiden.

Hier will die Erfindung Abhilfe schaffen. Die Erfindung löst die Aufgabe durch eine Vorrichtung bei der ein Gehäuse eine Öffnung aufweist, in der ein Spannfutter für die Kanülen angeordnet ist, das mit einem Antrieb zum Öffnen und Schließen des Spannfutters verbunden ist und das Gehäuse mit einer Schublade zur Aufnahme eines Auffangbehälters versehen ist, in den die Öffnung mündet.

Der Antrieb kann aus einem drehmomentbegrenzten Elektromotor bestehen und das Spannfutter als Schalter für die Stromzufuhr des Elektromotors ausgebildet sein. Alternativ kann die Öffnung mit einem solchen Schalter versehen sein. Das Gehäuse kann mit einem Sichtfenster und einem Saugfuß versehen und der Auffangbehälter mit einem Deckel verschließbar sein.

Im folgenden wird die Erfindung anhand der Figuren näher erläutert, die lediglich ein Ausführungsbeispiel darstellen. Es zeigt

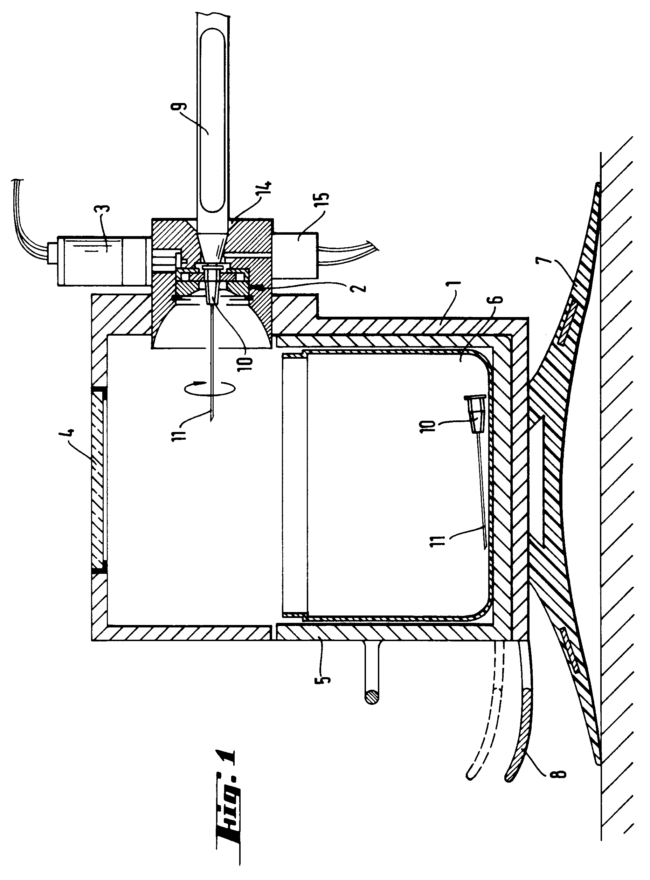

Figur 1 eine Seitenansicht der Vorrichtung geschnitten und

Figur 2 den Auffangbehälter.

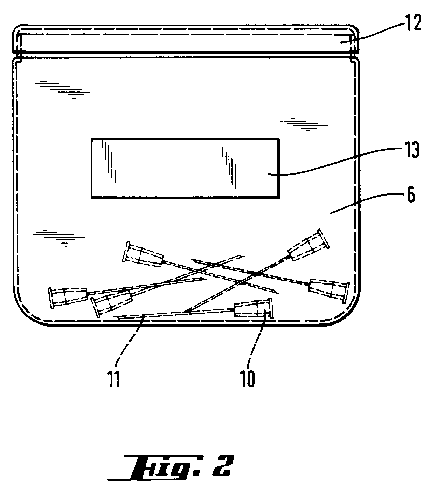

Die Vorrichtung besteht aus einem Gehäuse 1, das mit einer Öffnung 14 versehen ist. In der Öffnung 14 ist ein Spannfutter 2 für die Kanülen 11 bzw.
Verbindungsstück 10 angeordnet. Mit dem Spannfutter 2 ist ein Antrieb 3 zum Öffnen und Schließen des Spannfutters 2 verbunden. Der Antrieb kann aus einem drehmomentbegrenzten Elektromotor bestehen. Als Schalter für einen solchen Motor kann eine in der Öffnung 14 oder im Spannenfutter 2 angeordnete Lichtschranke bzw. ein in der Öffnung 14 angeordneter mechanischer Schalter 15 verwendet werden. Die Schalter werden durch Einschieben bzw. Herausziehen des Spritzenkörpers 9 betätigt. Durch Drehbewegung des Spannfutters 2 wird die Injektionskanüle 11 vom Spritzenkörper 9 abgedreht und fällt in den Auffangbehälter 6. Die einzelnen Bauelemente werden vom Gehäuse 1 umschlossen und durch ein Sichtfenster 4 kann überprüft werden, inwieweit der Auffangbehälter 6 mit Injektionskanülen 11 gefüllt ist. Die Vorrichtung besitzt eine Schublade 5, über die der Auffangbehälter 6 ausgetauscht werden kann. Gemäß Figur 2 wird der mit Injektionskanülen 11 gefüllte Auffangbehälter 6 mit einem Deckel 12 verschlossen. Der Entsorgungsinhalt kann auf einem Etikett 13 vermerkt werden. Die Vorrichtung kann mittels Saugfuß 7 und Befestigungshebel 8 auf einer entsprechenden Unterlage befestigt werden.

**Patentansprüche**

1. Vorrichtung zum Entsorgen von Injektionskanülen, dadurch gekennzeichnet, daß ein Gehäuse (1) eine Öffnung (14) aufweist, in der ein Spannfutter (2) für die Kanülen (11) angeordnet ist, wobei das Spannfutter (2) mit einem Antrieb (3) zum Öffnen und Schließen des Spannfutters (2) verbunden ist und das Gehäuse (1) mit einer Schublade (5) zur Aufnahme eines Auffangbehälters (6) versehen ist, in den die Öffnung (14) mündet.

2. Vorrichtung nach Anspruch 1, dadurch gekennzeichnet, daß der Antrieb (3) aus einem drehmomentbegrenzten Elektromotor besteht und das Spannfutter (2) als Schalter für die Stromzufuhr des Elektromotors ausgebildet ist.

3. Vorrichtung nach Anspruch 1, dadurch gekennzeichnet, daß der Antrieb (3) aus einem drehmomentbegrenzten Elektromotor besteht und die Öffnung (14) mit einem Schalter (15) für die Stromzufuhr des Elektromotors versehen ist.

4. Vorrichtung nach Anspruch 1, dadurch gekennzeichnet, daß das Gehäuse mit einem Sichtfenster (4) versehen ist.

5. Vorrichtung nach Anspruch 1, dadurch gekennzeichnet, daß das Gehäuse (1) mit einem Saugfuß (7) versehen ist.

6. Vorrichtung nach Anspruch 1, dadurch gekennzeichnet, daß der Auffangsbehälter (6) mit einem Deckel (12) verschließbar ist.

Fig. 1

EP 0 512 499 A1

**Fig. 2**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 989 307 (SHARPE ET AL)<br>* Spalte 3, Zeile 5 - Zeile 43 *<br>* Spalte 6, Zeile 29 - Zeile 37; Abbildungen *<br>--- | 1-6 | A61M5/32 |
| A | EP-A-0 313 212 (WILLOUGHBY)<br>* Zusammenfassung; Abbildungen *<br>--- | 1-6 | |
| A | US-A-4 807 344 (KELSON ET AL)<br>* Spalte 4, Zeile 6 - Zeile 17; Abbildungen *<br>--- | 1-6 | |
| A | DE-U-8 711 864 (HARTIG)<br>* Ansprüche 1,2; Abbildung 2 *<br><br>----- | 5 | |
| | | | **RECHERCHIERTE SACHGEBIETE (Int. Cl.5)**<br><br>A61M |

Der vorliegende Recherchenbericht wurde für alle Patentansprüche erstellt

| Recherchenort | Abschlußdatum der Recherche | Prüfer |
|---|---|---|
| DEN HAAG | 07 AUGUST 1992 | CLARKSON P. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
.........................................................................
& : Mitglied der gleichen Patentfamilie, übereinstimmendes
    Dokument

EPO FORM 1503 03.82 (P0403)